(19) Europäisches Patentamt
European Patent Office
Office européen des brevets

(11) Numéro de publication : **0 331 593 B1**

# (12) FASCICULE DE BREVET EUROPEEN

(45) Date de publication du fascicule du brevet : **25.11.92 Bulletin 92/48**

(51) Int. Cl.[5] : **C07C 45/49**, C07C 47/58, C07C 47/575, C07C 47/565, C07C 47/57

(21) Numéro de dépôt : **89420077.3**

(22) Date de dépôt : **28.02.89**

## (54) Procédé de préparation d'hydroxybenzaldehydes par hydrocarbonylation.

(30) Priorité : **01.03.88 FR 8802827**
**29.07.88 FR 8810536**

(43) Date de publication de la demande : **06.09.89 Bulletin 89/36**

(45) Mention de la délivrance du brevet : **25.11.92 Bulletin 92/48**

(84) Etats contractants désignés : **AT BE CH DE ES FR GB GR IT LI LU NL SE**

(56) Documents cités :
**EP-A- 0 109 606**
**EP-A- 0 244 328**
**US-A- 3 960 932**

(73) Titulaire : **RHONE-POULENC CHIMIE**
**25, quai Paul Doumer**
**F-92408 Courbevoie Cédex (FR)**

(72) Inventeur : **Fompeyrine, Patricia**
**5, avenue Valioud**
**F-69110 Sainte Foy-les-Lyon (FR)**
Inventeur : **Leconte, Philippe**
**30, rue Moenchsberg**
**F-68100 Mulhouse (FR)**
Inventeur : **Metz, François**
**22, rue de Condé**
**F-69002 Lyon (FR)**
Inventeur : **Perron, Robert**
**La Pecolière**
**F-69390 Charly (FR)**

(74) Mandataire : **Vignally, Noel et al**
**Rhône-Poulenc Interservices Service Brevets Chimie Centre de Recherches des Carrières**
**B.P. 62**
**F-69192 Saint-Fons Cédex (FR)**

EP 0 331 593 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait à un procédé de préparation d'hydroxybenzaldéhydes par hydrocarbonylation des halogénophénols correspondants.

Le brevet US 3 960 932 décrit un procédé général de préparation d'aldéhydes, par réaction d'halogénures d'aryle, de vinyle ou de composés hétérocycliques, avec un mélange de monoxyde de carbone et d'hydrogène, en présence d'une amine tertiaire et d'un catalyseur au palladium consistant en un complexe d'un dérivé du palladium divalent avec une phosphine, un phosphite ou une arsine ou en l'association d'un sel de palladium divalent ou de palladium métallique finement divisé avec un agent complexant du groupe des phosphines, des phosphites ou des arsines. Les halogénures d'aryles utilisés dans le procédé selon US 3 960 932 sont des bromures ou des iodures de phényle ou de naphtyle, non substitués ou substitués par des groupements alkyle, alcoxy, nitrile ou carboxylate d'alkyle.

Le brevet européen EP 109 606 propose d'augmenter la cinétique de la réaction d'hydrocarbonylation du précédent procédé, en opérant sous des pressions de 2 à 40 MPa (20 à 400 bar), à des températures de 80°C à 250°C et en utilisant des quantités importantes de phosphine ou de phosphite (2 à $10^5$ fois la quantité molaire de catalyseur).

Le document EP-A-0244 328 vise un procédé de synthèse d'aldéhydes aromatiques par hydrogénocarbonylation à partir de dérivés correspondant, mais ce document était silencieux sur les conditions supplémentaires à apporter au procédé pour que cette technique soit applicable aux halogéno phénols pour donner des hydroxyarylaldéhydes.

Il faut observer que ces procédés de l'art antérieur ne s'appliquent pas à l'hydrocarbonylation d'halogénophénols.

C'est précisément un objet de la présente invention de fournir un procédé de préparation d'hydroxybenzaldéhydes par réaction d'un halogénophénol avec un mélange monoxyde de carbone/hydrogène, en présence d'une amine tertiaire, d'un catalyseur à base d'un métal noble et d'une phosphine.

Plus particulièrement, il s'agit d'un procédé de préparation d'un hydroxybenzaldéhyde de formule générale (I) :

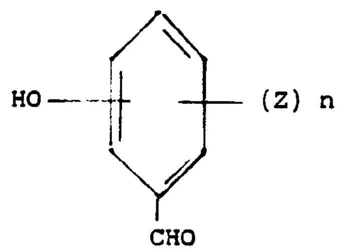

(I)

dans laquelle
- n représente 0, 1 ou 2
- Z représente un groupement électron-donneur ou un groupement électron-attracteur

par réaction d'un halogénophénol de formule générale (II)

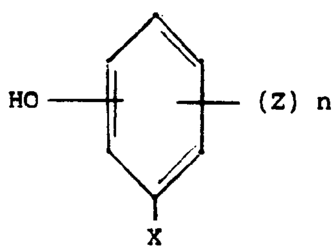

(II)

dans laquelle
- X représente un atome de brome ou un atome d'iode ;
- Z a les significations indiquées précédemment ;
- n représente 0 ou 1 ou 2 ;

avec un mélange monoxyde de carbone/hydrogène, en présence d'un catalyseur à base d'un métal noble, d'une amine tertiaire, d'une triarylphosphine ou d'une diarylalkylphosphine, caractérisé en ce que l'amine tertiaire est telle que le pKa de son acide conjugué soit, d'une part inférieur ou égal ou pKa de l'halogénophénol de formule (II) et, d'autre part, supérieur à celui de la phosphine.

Le pKa dans l'eau (généralement à 25°C) de l'acide conjugué de l'amine tertiaire est indique dans des tables que l'on trouve dans la littérature.

Le pKa dans l'eau de la phosphine est également indiqué dans des tables que l'on trouve dans la littérature.

Le pKa de l'halogénophénol de formule (II) est déterminé selon la méthode E3bg de l'IUPAC intitulée "IONISATION CONSTANTS OF ORGANIC ACIDS IN AQUEOUS SOLUTIONS" (editeur Pergamon Press ; 1979).

Il a donc été trouvé que pour pouvoir hydrocarbonyler un halogénophénol de formule (II) afin de préparer un hydroxybenzaldéhyde de formule (I), le pKa de l'acide conjugué de l'amine tertiaire utilisée doit être compris entre celui de la phosphine utilisée et celui de l'halogénophénol (II) mis en oeuvre.

Lorsque l'amine tertiaire utilisée ne répond pas à cette condition, il y a essentiellement formation d'un composé polymérique, mais pratiquement pas d'hydroxybenzaldéhyde.

Par triarylphosphine et diarylalkylphosphine, on entend dans le présent texte une phosphine de formule générale (III) :

$$\begin{array}{l} P-(Ar)_2 \\ | \\ R \end{array} \qquad (III)$$

dans laquelle :

- les symboles Ar identiques ou différents représentent :
  - * un radical phényle
  - * un radical phényle substitué par un ou plusieurs radicaux ou atomes tels que alkyle ayant 1 à 4 atomes de carbone ; alcoxy ayant 1 à 4 atomes de carbone ; trifluorométhyle ; diméthylamino ; diéthylamino ; chlore ou fluor ;
  - * un radical naphtyle
  - * un radical naphtyle substitué par un ou plusieurs radicaux ou atomes tels que alkyle ayant 1 à 4 atomes de carbone ; alcoxy ayant 1 à 4 atomes de carbone ; trifluorométhyle ; diméthylamino ; diéthylamino ; chlore ou fluor ;
- R représente :
  - * un radical alkyle ayant 1 à 12 atomes de carbone
  - * les radicaux représentés par Ar

Parmi les phosphines de formule (III), on utilisera de préférence celles pour lesquelles :

- les symboles Ar identiques ou différents représentent
  - * un radical phényle
  - * un radical phényle portant 1 ou 2 substituants choisis parmi les radicaux méthoxy, éthoxy, méthyle, éthyle, trifluorométhyle, diméthylamino et les atomes de chlore ou de fluor
  - * un radical naphtyle
- R représente un radical alkyle ayant 1 à 4 atomes de carbone ou l'un des radicaux représentés par Ar.

De préférence les substituants que peuvent comporter les phosphines de formule (III) sur les radicaux Ar, sont situés en position para et/ou méta par rapport au carbone du cycle lié au phosphore.

Parmi les phosphines de formule (III) que l'on peut utiliser dans le cadre de l'invention, on peut citer à titre d'exemples :

- la triphényl-phosphine,
- la tris(paraméthoxyphényl)phosphine,
- la tris(paraméthylphényl)phosphine,
- la tris(métaméthylphényl)phosphine,
- la diphényl-méthyl-phosphine,
- la diphényl-éthyl-phosphine,
- la trinaphtyl-phosphine.

Les halogénophénols de formule (II) auxquels s'applique la présente invention sont plus particulièrement ceux pour lesquels n représente 0 ou 1 ou 2, le symbole Z représente un radical hydroxyle, un atome de brome, un atome d'iode, un radical alkyle, un radical alcoxy, un radical alkyle ou alcoxy substitué par un ou plusieurs atomes de chlore ou de fluor, un radical cycloalkyle, un radical phényle, un radical cycloalcoxy, un radical phénoxy, un radical alcoxycarbonyle, un radical cycloalcoxycarbonyle, un radical phénoxycarbonyle, un radical alkylcarbonyloxy, un radical cycloalkylcarbonyloxy, un radical phénylcarbonyloxy, l'un des radicaux précédents substitué par un ou plusieurs atomes de fluor et/ou de chlore ou groupes nitrile et le symbole X représente un atome de brome ou un atome d'iode.

Plus particulièrement dans les formules (I) et (II),
- n représente 0 ou 1 ou 2,
- X représente un atome de brome,
- Z représente :
. un radical hydroxyle ;
. un atome de brome ;
. un radical alkyle linéaire ou ramifié de 1 à 20 atomes de carbone ou un radical alkyle substitué par un ou plusieurs atomes de fluor et/ou de chlore, tels que par exemple les radicaux méthyle, éthyle, propyles, butyles, pentyles, hexyles, octyles, décyles, trifluorométhyle, difluorochlorométhyle, trichlorométhyle ; de préférence un radical alkyle inférieur, c'est-à-dire ayant 1 à 4 atomes de carbone ou un radical alkyle inférieur substitué par 1 à 3 atomes de fluor et/ou de chlore ;
. un radical alcoxy linéaire ou ramifié ayant 1 à 20 atomes de carbone ou un radical alcoxy substitué par un ou plusieurs atomes de fluor et/ou de chlore ; de préférence un radical alcoxy inférieur (ayant 1 à 4 atomes de carbone) ou un radical alcoxy inférieur substitué par 1 à 3 atomes de fluor et/ou de chlore, tel que par exemple les radicaux méthoxy, éthoxy, isopropoxy, difluorochlorométhoxy ou trichlorométhoxy ;
. un radical cyclopentyle, cyclohexyle ou cyclooctyle ;
. un radical phényle ou un radical phényle substitué par 1 à 3 radicaux alkyles ou alcoxy inférieurs, tel que les radicaux xylyles, toluyles, méthoxyphényles, éthoxyphényles ;
. un radical alcoxycarbonyle ayant 2 à 11 atomes de carbone et de préférence 2 à 5 atomes de carbone tels que par exemple les radicaux méthoxycarbonyle, éthoxycarbonyle, propoxycarbonyle et butoxycarbonyle ;
. un radical alcoxycarbonylalkyle dont la partie alcoxycarbonyle est telle que définie précédemment et la partie alkyle comporte 1 à 4 atomes de carbone ;
. un radical cyclopentyloxycarbonyle ou cyclohexyloxycarbonyle ;
. un radical phénoxycarbonyle ou méthylphénoxycarbonyle ;
. un radical alkylcarbonyloxy ayant 2 à 11 atomes de carbone et de préférence 2 à 5 atomes de carbone, tel que par exemple les radicaux acétoxy, propionyloxy, butyryloxy ;
. un radical cyclopentanoyloxy ou cyclohexanoyloxy ;
. un radical benzoyloxy, méthylbenzoyloxy ou diméthylbenzoyloxy.

Dans la formule (I), Z peut en outre représenter un groupement aldéhyde.

Comme exemples spécifiques d'hydroxybenzaldéhydes de formule (I) que l'on peut obtenir par le procédé selon l'invention, on peut citer de manière non limitative l'hydroxy-4 benzaldéhyde, l'hydroxy-2 benzaldéhyde, la vanilline (ou hydroxy-4 méthoxy-3 benzaldéhyde), l'hydroxy-2 méthoxy-5 benzaldéhyde, l'hydroxy-2 méthoxy-3 benzaldéhyde, l'hydroxy-4 éthoxy-3 benzaldéhyde, l'hydroxy-2 éthoxy-3 benzaldéhyde, l'hydroxy-2 éthoxy-5 benzaldéhyde, le diméthoxy-3,5 hydroxy-4 benzaldéhyde, le dihydroxy-3,4 benzaldéhyde, le dihydroxy-2,5 benzaldéhyde, le dihydroxy-2,3 benzaldéhyde, le dibromo-3,5 hydroxy-4 benzaldéhyde, le bromo-3 hydroxy-4 benzaldéhyde, le formyl-3 hydroxy-4 benzaldéhyde, le bromo-3 hydroxy-4 formyl-5 benzaldéhyde, le diformyl-3,5 hydroxy-4 benzaldéhyde.

Comme exemples spécifiques d'halogénophénols de formule (II) que l'on peut mettre en oeuvre dans le procédé selon l'invention, on peut citer non limitativement le bromo-4 phénol,le bromo-2 phénol, le bromo-4 méthoxy-2 phénol, le bromo-2 méthoxy-4 phénol, le bromo-6 méthoxy-2 phénol, le bromo-4 éthoxy-2 phénol, le bromo-2 éthoxy-4 phénol, le bromo-6 éthoxy-2 phénol, le bromo-4 diméthoxy-2,6 phénol, le bromo-4 dihydroxy-1,2 benzène, le bromo-2 dihydroxy-1,4 benzène, le bromo-3 dihydroxy-1,2 benzène, le dibromo-2,4 phénol, le tribromo-2,4,6 phénol.

A titre de catalyseurs, on peut utiliser pour la mise en oeuvre du procédé selon l'invention, un métal noble finement divisé, du groupe VIII de la classification périodique des éléments tel que le palladium, le rhodium et l'iridium, ou leurs sels d'acides minéraux ou organiques.

Les dérivés du palladium conviennent tout particulièrement bien au procédé de l'invention.

Comme exemples spécifiques de dérivés du palladium, on peut citer les carboxylates tels que notamment les acétates, propionates, butyrates ou benzoates de palladium (II), le chlorure palladeux.

On peut également utiliser des complexes des sels minéraux ou organiques du palladium avec la phosphine mise en oeuvre.

Dans ce dernier cas, ce complexe est généralement réalisé in situ entre le dérivé du palladium et la phosphine présente. Mais on peut également préparer ledit complexe extemporanément et l'introduire dans le milieu réactionnel. On peut alors rajouter ou non une quantité supplémentaire de phosphine libre.

La quantité de catalyseur exprimée en moles d'atomes de métal ou en moles de dérivé métallique par mole d'halogènophénol de formule (II) peut varier dans de larges limites.

Ainsi, elles peut être comprise entre $10^{-5}$ et $10^{-1}$ mole/mole et de préférence entre $10^{-4}$ et $10^{-2}$ mole/mole.

La quantité de phosphine libre et/ou sous forme de complexe avec le catalyseur, est telle que le rapport molaire phosphine/métal noble du catalyseur soit au moins égal à 2.

Le rapport phosphine/métal noble peut atteindre des valeurs aussi élevées que 10.000.

Un rapport phosphine/métal noble compris entre 4 et 1.000 est en général très convenable.

L'amine tertiaire utilisée dans le présent procédé peut être une amine de formule générale (IV)

$$N - (R_1)_3 \qquad (IV)$$

dans laquelle :

- les radicaux $R_1$, identiques ou différents, représentent des restes hydrocarbonés ayant de 1 à 20 atomes de carbone, tels que des radicaux alkyle, cycloalkyle, aryle ou hétérocyclique ;
- deux radicaux $R_1$ forment ensemble et avec l'atome d'azote un hétérocyclique ayant 4 à 6 atomes.

Plus particulièrement :

- les symboles $R_1$ représentent un radical alkyle ayant 1 à 10 atomes de carbone et de préférence 1 à 4 atomes de carbone ou un radical cyclopentyle ou cyclohexyle ou un radical pyridinyle ;
- deux radicaux $R_1$ forment ensemble et avec l'atome d'azote un cycle pipéridine ou pyrrolidine.

A titre d'exemples de telles amines, on peut citer la triéthylamine, la tri-n-propylamine, la tri-n-butylamine, la méthyldibutylamine, la méthyldicyclohexylamine, l'ethyldiisopropylamine, la N,N-diéthylcyclohexylamine, la diméthylamino-4 pyridine, la N-méthylpipéridine, la N-méthylpyrrolidine.

L'amine tertiaire peut être aussi une amine tertiaire hétérocyclique telle que par exemple la pyridine, la béta-picoline, l'alpha-picoline, la gamma-picoline, la diméthyl-2,6 pyridine, la diméthyl-3,4 pyridine, la diméthyl-2,4 pyridine, la quinoléine, l'isoquinoléine, la phtalazine, la naphtyridine-1,8, la quinoxaline, la quinazoline, la cinnoline, la ptéridine.

Lorsque l'on met en oeuvre un halogénophénol comme le bromo-2 phénol, le bromo-4 phénol, le bromo-4 méthoxy-2 phénol ou le bromo-4 éthoxy-2 phénol qui conduisent à des hydroxybenzaldéhydes très importants et dont le pKa (à 25°C) est de 9,5 pour le bromo-4 phénol et le bromo-4 méthoxy-2 phénol, proche de cette valeur pour le bromo-4 éthoxy-2 phénol et de 8,55 pour le bromo-2 phénol, les amines tertiaires hétérocycliques conviennent généralement bien. En effet le pKa de leur acide conjugué est le plus souvent inférieur à 9,5, tandis que les amines tertiaires de formule (IV) telles que la triéthylamine qui ont un pKa de leur acide conjugué plus élevé que 9,5, ne peuvent pas être utilisées. Elles conduisent essentiellement à des composés polymériques.

La quantité d'amine tertiaire utilisée doit être suffisante pour neutraliser l'hydracide libéré par la réaction.

En outre, la concentration en amine tertiaire dans le milieu doit être au moins de 2 moles par litre pendant la durée de la réaction.

Il n'y a pas de limite supérieure critique à la quantité d'amine tertiaire, qui peut donc être utilisée en large excès par rapport à la quantité théoriquement nécessaire à la neutralisation de l'hydracide formé.

Pour maintenir la concentration en amine tertiaire, au moins égale aux valeurs limites indiquées précédemment, pendant toute la durée de la réaction, la quantite d'amine engagée doit être calculée de telle façon qu'en fin de réaction, la concentration soit au moins égale à ces valeurs. On peut également ajouter une quantité d'amine tertiaire supplémentaire au fur et à mesure du déroulement de la réaction, afin de compenser la quantité d'amine consommée par la neutralisation de l'hydracide.

On peut utiliser des mélanges $CO/H_2$ présentant différents rapports molaires des deux gaz. Généralement, le rapport molaire $CO/H_2$ varie entre 0,1 et 10.

La pression sous laquelle est mis en oeuvre le procédé varie aussi très largement. Généralement, elle va de 0,1 à 30 MPa (1 à 300 bar) et de préférence de 1 à 15 MPa (10 à 150 bar).

Le procédé selon l'invention est conduit en phase liquide.

On peut faire appel à un solvant inerte dans les conditions de la réaction d'hydrocarbonylation. Ainsi, on peut utiliser des hydrocarbures aliphatiques ou cycloaliphatiques saturés tels que l'hexane ou le cyclohexane, ou aromatiques tels que le benzène, le toluène, les xylènes ; des esters tels que le benzoate de méthyle, le téréphtalate de méthyle, l'adipate de méthyle, le phtalate de dibutyle ; des esters ou éthers de polyols tels que le diacétate de tétraéthylène glycol ; des éthers cycliques tels que le tétrahydrofuranne ou le dioxanne.

La concentration de l'halogénophénol de formule (II) mis en oeuvre dans le solvant peut varier dans de très larges limites, jusqu'à la saturation dans les conditions opératoires. Généralement, il n'est pas économiquement intéressant d'utiliser moins de 5 % en poids d'halogénophénol par volume de solvant.

De manière générale, la concentration en poids d'halogénophénol par volume de solvant est comprise entre 5 % et 50 % et de préférence entre 10 % et 40 % .

En pratique, on peut mettre en oeuvre le procédé selon l'invention en introduisant dans un autoclave inerte l'halogénophénol de formule (II), l'amine tertiaire, le catalyseur, la phosphine et le solvant, puis, après les purges habituelles, en alimentant l'autoclave avec une pression adéquate d'un mélange $CO/H_2$ ; le contenu de

l'autoclave est ensuite porté sous agitation à la température convenable jusqu'à ce que l'absorption cesse. La pression dans l'autoclave peut être maintenue constante pendant la durée de la réaction grâce à une réserve de mélange gazeux, qui l'alimente à la pression choisie.

En fin d'essai, l'autoclave est refroidi et dégazé. Le mélange réactionnel est récupéré.

Un mode de traitement très simple consiste à ajouter au mélange réactionnel une solution aqueuse d'hydroxyde de métal alcalin.

Après agitation, puis décantation, on obtient ainsi une phase aqueuse et une phase organique. La phase organique contient essentiellement le catalyseur, la phosphine et au moins une partie de l'amine tertiaire. Cette solution organique peut facilement être recyclée dans une nouvelle réaction d'hydrocarbonylation, après ajout d'une nouvelle charge de l'halogénophénol et d'un éventuel complément en amine tertiaire.

La phase aqueuse contient essentiellement l'hydroxybenzaldéhyde formé sous forme du phénate de métal alcalin, ainsi que les éventuels sous-produits et l'halogénophénol éventuellement non transformé, également sous forme de dérivés de métal alcalin.

Une simple acidification et soit une recristallisation lorsque le produit est solide, soit une distillation lorsque le produit est liquide, permettent de récupérer l'hydroxybenzaldéhyde pur.

Le procédé peut être mis en oeuvre en discontinu ou en continu comme indiqué précédemment en recyclant le catalyseur, la phosphine et l'amine tertiaire.

Les exemples qui suivent illustrent l'invention.

Exemples 1 à 6 et essais comparatifs A et B

Dans un autoclave de 125 $cm^3$, en alliage de marque commerciale HASTELLOY B2, muni d'un dispositif de chauffage et d'une agitation on charge :

- 10,15 g (50 mmol) de bromo-4 méthoxy-2 phénol
- 0,22 g ( 1 mmol) de diacétate de palladium
- 1,51 g ( 5 mmol) de triphénylphosphine (pKa à 25°C : 2,73)
- 110 mmol d'amine tertiaire (indiquée dans le tableau I ci-après)
- 17,5 $cm^3$ de toluène.

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C ; on ajuste la pression $CO/H_2$ à 3 MPa (30 bar), puis la température est portée à 150°C.

On maintient la température à 150°C et la pression à 3 MPa jusqu'à la fin d'absorption du mélange $CO/H_2$.

L'autoclave est alors refroidi, dégazé.

Après prélèvement d'un échantillon pour dosage par chromatographie liquide, on ajoute au mélange réactionnel 40 $cm^3$ d'une solution aqueuse de soude (6 g de soude) et on agite l'autoclave à température ambiante pendant 1 heure.

On sépare la phase aqueuse par décantation, on l'acidifie par HCl jusqu'à pH 1 et on l'extrait par 3 fois 100 $cm^3$ d'éther éthylique.

La solution éthérée obtenue est traitée par 2 fois 50 $cm^3$ d'une solution aqueuse d'hydrogénocarbonate de sodium à 20 %.

La solution éthérée est ensuite décantée et l'éther est évaporé.

Le solide brun obtenu est recristallisé dans le toluène, puis dans l'eau pour conduire à la vanilline pure.

Le tableau I ci-après reprend pour chaque essai les indications concernant l'amine tertiaire utilisée, la durée, la vitesse de réaction exprimée en $MPa.h^{-1}$, le taux de transformation (TT %) du bromo-4 méthoxy-2 phénol (BMPH), le rendement (RT %) en hydroxy-4 méthoxy-3 benzaldéhyde (vanilline) (HMBZ) par rapport au bromo-4 méthoxy-2 phénol transformé, les RT % en gaïacol et éventuellement en acide hydroxy-4 méthoxy-3 benzoïque formés.

La différence à 100 % de la somme des RT indiqués précedemment correspond à la formation d'un composé polymérique de structure :

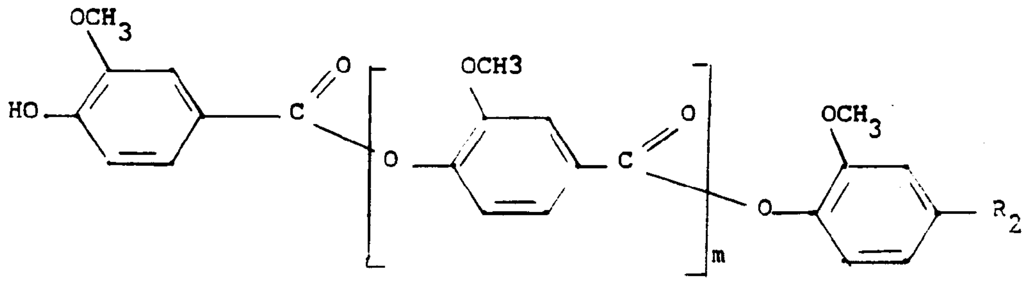

m étant égal ou supérieur à 1 et $R_2$ représentant CHO, H ou Br.

On note que :

- lorsque le pKa de l'acide conjugué de l'amine tertiaire est supérieur à celui du bromo-4 méthoxy-2 phénol mis en oeuvre, il n'y a pas de formation d'aldéhyde (essai A)
- lorsque le pKa de l'acide conjugué de l'amine tertiaire est inférieur à celui de la triphénylphosphine, le rendement en aldéhyde est très faible (essai B)
- lorsque le pKa de l'acide conjugué de l'amine tertiaire est sensiblement égal à celui du bromo-4 méthoxy-2 phénol, le rendement en aldéhyde est faible (exemple 6).

**TABLEAU I**

| Essais | Amine tertiaire | pKa * | Durée | Vitesse en MPa.h-1 | TT % en BMPH ** | RT % en HMBZ ** | RT % en gaïacol | RT % en HMBQ ** |
|---|---|---|---|---|---|---|---|---|
| Exemple 1 | Pyridine | 5,25 | 2 h 50 | 0,9 | 98 | 26 | 20 | 3,2 |
| Exemple 2 | Gamma Picoline | 6,02 | 2 h 20 | 1,2 | 99 | 35 | 17 | 5,7 |
| Exemple 3 | Diméthyl-3,4 Pyridine | 6,57 | 2 h 05 | 1,4 | 100 | 31 | 6,8 | 2,3 |
| Exemple 4 | Diméthyl-2,6 Pyridine | 6,73 | 4 h 25 | 0,63 | 100 | 28 | 4,0 | 8,1 |
| Exemple 5 | Diméthyl-2,4 Pyridine | 6,99 | 2 h 55 | 1,3 | 100 | 46 | 9,0 | 5,7 |
| Exemple 6 | Diméthylamino-4 Pyridine | 9,5 | 18 min | 7,5 | 100 | 8,0 | 1,5 | 0,2 |
| Essai comparatif A | Triéthyl amine | 11,1 | 20 min | 10 | 100 | 0 | 0 | 0 |
| Essai comparatif B | Pyrazine | 0,65 | 6 h 25 | 0,3 | 88 | 5,9 | 14,0 | 4,1 |

* pka del'acide conjugué de l'amine tertiaire

** BMPH = bromo-4 méthoxy-2 phénol
HMBZ = hydroxy-4 méthoxy-3 benzaldéhyde (vanilline)
HMBQ = acide hydroxy-4 méthoxy-3 benzoïque

Exemples 7 à 10 et essai comparatif D

On répète les exemples 1 à 6 dans les mêmes conditions et avec les mêmes rapports de réactifs, mais en mettant en oeuvre du bromo-2 phénol comme halogénophénol de formule (II).

Le bromo-2 phénol a un pKa à 25°C de 8,55.

Les amines tertiaires utilisées sont indiquées dans le tableau II ci-après, qui rassemble les résultats obtenus pour chaque exemple ainsi que pour l'essai comparatif D (effectué avec la triéthylamine) qui n'entre pas dans le cadre de l'invention.

Le traitement des essais et le dosage des produits obtenus sont les mêmes que pour les exemples 1 à 6.

La différence à 100 % de la somme des RT indiqués dans le tableau II correspond à un composé polymérique ayant une structure du type de celle indiquée dans les exemples 1 à 6.

On note que lorsque le pKa de l'acide conjugué de l'amine tertiaire est supérieur à celui du bromo-2 phénol mis en oeuvre, le rendement en aldéhyde est très faible (essai D).

TABLEAU II

| Essais | Amine tertiaire | pKa * | Durée | TT % du B2PH ** | RT % en H2BZ ** | RT % en phénol | RT % en H2BQ ** |
|---|---|---|---|---|---|---|---|
| Exemple 7 | Pyridine | 5,25 | 2 h 50 | 74 | 31 | 51 | 0 |
| Exemple 8 | Diméthyl-3,4 Pyridine | 6,57 | 2 h 05 | 83 | 36 | 37 | 2,0 |
| Exemple 9 | Diméthyl-2,6 Pyridine | 6,73 | 4 h 25 | 86 | 30 | 46 | 5,0 |
| Exemple 10 | Diméthyl-2,4 Pyridine | 6,99 | 2 h 55 | 85 | 38 | 37 | 5,7 |
| Essai comparatif D | Triéthyl amine | 11,1 | 20 min | 100 | 6,0 | 2,0 | 7,0 |

* pka de l'acide conjugué de l'amine tertiaire

** B2PH = bromo-2 phénol
H2BZ = hydroxy-2 benzaldéhyde (aldéhyde salicylique)
H2BQ = acide hydroxy-2 benzoïque (acide salicylique)

Exemples 11 à 13 et essais comparatifs E et F

On répète les exemples 1 à 6 dans les mêmes conditions et avec les mêmes rapports de réactifs, mais en mettant en oeuvre du bromo-4 phénol comme halogénophénol de formule (II).

Le bromo-4 phénol a un pKa à 25°C de 9,5.

Les amines tertiaires utilisées sont indiquées dans le tableau III ci-après, qui rassemble les résultats obtenus pour chaque exemple ainsi que pour les essais comparatifs E et F (effectués avec la pyrazine et la triéthylamine) qui n'entrent pas dans le cadre de l'invention.

Le traitement des essais et le dosage des produits obtenus sont les mêmes que pour les exemples 1 à 6.

La différence à 100 % de la somme des RT indiqués dans le tableau III correspond à un composé polymérique ayant une structure du type de celle indiquée dans les exemples 1 à 6.

On note que :

- lorsque le pKa de l'acide conjugué de l'amine tertiaire est inférieur à celui de la phosphine mise en oeuvre, le rendement en aldéhyde est très faible (essai E)
- lorsque le pKa de l'acide conjugué de l'amine tertiaire est supérieur à celui du bromo-4 phénol mis en oeuvre, le rendement en aldéhyde est très faible (essai F).

TABLEAU III

| Essais | Amine tertiaire | pKa * | Durée | TT % du B4PH ** | RT % en H4BZ ** | RT % en phénol | RT % en H4BQ ** |
|---|---|---|---|---|---|---|---|
| Exemple 11 | Pyridine | 5,25 | 2 h 20 | 87 | 22 | 20 | 0 |
| Exemple 12 | Diméthyl-2,4 Pyridine | 6,99 | 2 h 50 | 95 | 41 | 5,0 | 5,0 |
| Exemple 13 | Diméthylamino-4 Pyridine | 9,5 | 20 min | 99 | 7,0 | 0,8 | 0,5 |
| Essai comparatif E | Pyrazine | 0,65 | 4 h 45 | 79 | 0,4 | 17,0 | 13,0 |
| Essai comparatif F | Triéthyl amine | 11,1 | 20 min | 99 | 2,0 | 2,0 | 0 |

* pka de l'acide conjugué de l'amine tertiaire

** B4PH = bromo-4 phénol
H4BZ = hydroxy-4 benzaldéhyde
H4BQ = acide hydroxy-4 benzoïque

Exemples 14 à 16 et essais comparatifs G et H

Dans un autoclave de 125 $cm^3$, en alliage de marque commerciale HASTELLOY B2, muni d'un dispositif de chauffage et d'une agitation on charge :

- 10,15 g (50 mmol) de bromo-4 méthoxy-2 phénol
- 0,22 g ( 1 mmol) de diacétate de palladium
- 5 mmol de tris(méthyl-3 phényl)phosphine (pKa à 25°C : 3,3)
- 110 mmol d'amine tertiaire (indiquée dans le tableau IV ci-après)
- 17,5 $cm^3$ de toluène.

L'autoclave est fermé et purgé à l'aide d'un mélange équimoléculaire de CO et de $H_2$.

On charge ensuite 0,1 MPa (1 bar) de pression de ce mélange $CO/H_2$ ; on porte sous agitation le contenu de l'autoclave à 100°C; on ajuste la pression $CO/H_2$ à 3 MPa (30 bar), puis la température est portée à 150°C.

On maintient la température à 150°C et la pression à 3 MPa jusqu'à la fin d'absorption du mélange $CO/H_2$.

L'autoclave est alors refroidi, dégazé.

Après prélèvement d'un échantillon pour dosage par chromatographie liquide, on ajoute au mélange réactionnel 40 $cm^3$ d'une solution aqueuse de soude (6 g de soude) et on agite l'autoclave à température ambiante pendant 1 heure.

On sépare la phase aqueuse par décantation, on l'acidifie par HCl jusqu'à pH 1 et on l'extrait par 3 fois 100 $cm^3$ d'éther éthylique.

La solution éthérée obtenue est traitée par 2 fois 50 $cm^3$ d'une solution aqueuse d'hydrogénocarbonate de sodium à 20 %.

La solution éthérée est ensuite décantée et l'éther est évaporé.

Le solide brun obtenu est recristallisé dans le toluène, puis dans l'eau pour conduire à la vanilline pure.

Le tableau IV ci-après reprend pour chaque essai les indications concernant l'amine tertiaire utilisée, la durée, le taux de transformation (TT %) du bromo-4 méthoxy-2 phénol (BMPH), le rendement (RT %) en hydroxy-4 méthoxy-3 benzaldéhyde (vanilline) (HMBZ) par rapport au bromo-4 méthoxy-2 phénol transformé, les RT % en gaïacol et éventuellement en acide hydroxy-4 méthoxy-3 benzoïque formés.

La différence à 100 % de la somme des RT indiqués précedemment correspond à la formation d'un composé polymérique de structure :

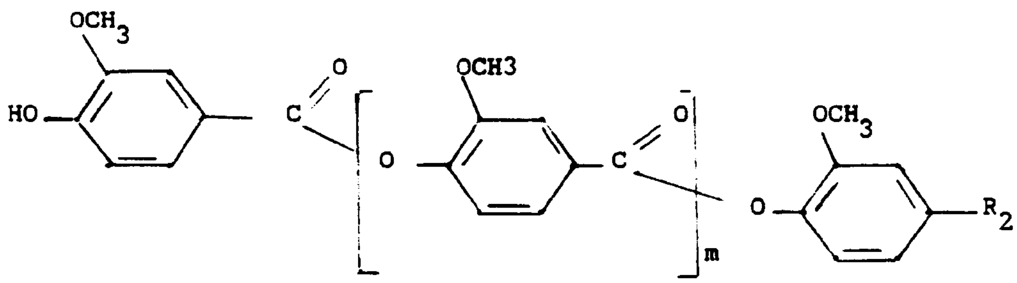

m étant égal ou supérieur à 1 et $R_2$ représentant CHO, H ou Br.

On note que:

- lorsque le pKa de l'acide conjugué de l'amine tertiaire est supérieur à celui du bromo-4 méthoxy-2 phénol mis en oeuvre, ou lorsque le pKa de l'acide conjugué de l'amine tertiaire est inférieur à celui de la phosphine, le rendement en aldéhyde est très faible (essais G et H)
- lorsque le pKa de l'acide conjugué de l'amine tertiaire est sensiblement égal à celui du bromo-4 méthoxy-2 phénol, le rendement en aldéhyde est faible (exemple 16).

TABLEAU IV

| Essais | Amine tertiaire | pKa * | Durée | TT % du BMPH ** | RT % en HMBZ ** | RT % en gaïacol | RT % en HMBQ ** |
|---|---|---|---|---|---|---|---|
| Exemple 14 | Pyridine | 5,25 | 3 h 30 | 99,6 | 31 | 16 | 4,0 |
| Exemple 15 | Diméthyl-2,4 Pyridine | 6,99 | 2 h 50 | 99,5 | 43 | 7,0 | 7,0 |
| Exemple 16 | Diméthylamino-4 Pyridine | 9,5 | 25 min | 100 | 9,0 | 1,0 | 0 |
| Essai comparatif G | Triéthyl amine | 11,1 | 15 min | 100 | 4,0 | 3,0 | 0,4 |
| Essai comparatif H | Pyrazine | 0,65 | 11 h | 98 | 0,3 | 27,0 | 6,0 |

* pka de l'acide conjugué de l'amine tertiaire

** BMPH = bromo-4 méthoxy-2 phénol
HMBZ = hydroxy-4 méthoxy-3 benzaldéhyde (vanilline)
HMBQ = acide hydroxy-4 méthoxy-3 benzoïque

## Revendications

1. Procédé de préparation d'un hydroxybenzaldéhyde de formule générale (I) :

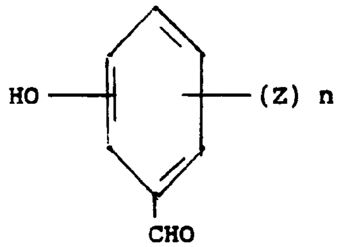

(I)

dans laquelle

- n représente 0 ou 1 ou 2
- Z représente un groupement électron-donneur ou un groupement électron-attracteur

par réaction d'un halogénophénol de formule générale (II)

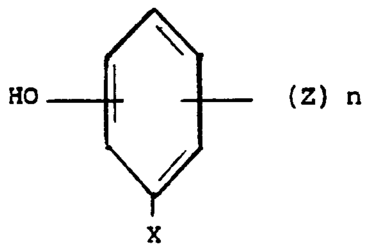

(II)

dans laquelle

- X représente un atome de brome ou un atome d'iode ;
- Z a les significations indiquées précédemment ;
- n représente 0 ou 1 ou 2 ;

avec un mélange monoxyde de carbone/hydrogène, en présence d'un catalyseur à base d'un métal noble, d'une amine tertiaire, d'une triarylphosphine ou d'une diarylalkylphosphine, caractérisé en ce que l'amine tertiaire est telle que le pKa de son acide conjugué soit, d'une part inférieur ou égal au pKa de l'halogénophénol de formule (II) et, d'autre part, supérieur à celui de la phosphine.

2. Procédé selon la revendication 1 caractérisé en ce que l'on met en oeuvre un halogénophénol de formule (II) dans laquelle :
   - n représente 0 ou 1 ou 2 ;
   - le symbole Z représente un radical hydroxyle, un atome de brome, un atome d'iode, un radical alkyle, un radical alcoxy, un radical alkyle ou alcoxy substitué par un ou plusieurs atomes de chlore ou de fluor, un radical cycloalkyle, un radical phényle, un radical cycloalkoxy, un radical phénoxy, un radical alcoxycarbonyle, un radical cycloalcoxycarbonyle, un radical phénoxycarbonyle, un radical alkylcarbonyloxy, un radical cycloalkylcarbonyloxy, un radical phénylcarbonyloxy, l'un des radicaux précédents substitué par un ou plusieurs atomes de fluor et/ou de chlore ou groupes nitrile ;
   - le symbole X représente un atome de brome ou un atome d'iode.

3. Procédé selon l'une des revendications 1 ou 2, caractérisé en ce que l'on met en oeuvre un halogénophénol de formule (II) dans laquelle :
   - n représente 0 ou 1 ou 2 ;
   - X représente un atome de brome ;
   - Z représente :
     . un radical hydroxyle ;
     . un atome de brome ;
     . un radical alkyle linéaire ou ramifié de 1 à 20 atomes de carbone ou un radical alkyle substitué par un ou plusieurs atomes de fluor et/ou de chlore ; de préférence un radical alkyle inférieur, c'est-à-dire ayant 1 à 4 atomes de carbone ou un radical alkyle inférieur substitué par 1 à 3 atomes de fluor et/ou de chlore ;
     . un radical alcoxy linéaire ou ramifié ayant 1 à 20 atomes de carbone ou un radical alcoxy substitué par un ou plusieurs atomes de fluor et/ou de chlore ; de préférence un radical alcoxy inférieur (ayant

1 à 4 atomes de carbone) ou un radical alcoxy inférieur substitué par 1 à 3 atomes de fluor et/ou de chlore ;
. un radical cyclopentyle, cyclohexyle ou cyclooctyle ;
. un radical phényle ou un radical phényle substitué par 1 à 3 radicaux alkyles ou alcoxy inférieurs ;
. un radical alcoxycarbonyle ayant 2 à 11 atomes de carbone et de préférence 2 à 5 atomes de carbone ;
. un radical alcoxycarbonylalkyle dont la partie alcoxycarbonyle est telle que définie précédemment et la partie alkyle comporte 1 à 4 atomes de carbone ;
. un radical cyclopentyloxycarbonyle ou cyclohexyloxycarbonyle ;
. un radical phénoxycarbonyle ou méthylphénoxycarbonyle ;
. un radical alkylcarbonyloxy ayant 2 à 11 atomes de carbone et de préférence 2 à 5 atomes de carbone ;
. un radical cyclopentanoyloxy ou cyclohexanoyloxy ;
. un radical benzoyloxy, méthylbenzoyloxy ou diméthylbenzoyloxy.

4. Procédé selon l'une des revendications 1 à 3, caractérisé en ce que l'halogénophénol de formule (II) est le bromo-4 phénol, le bromo-2 phénol, le bromo-4 méthoxy-2 phénol, le bromo-2 méthoxy-4 phénol, le bromo-6 méthoxy-2 phénol, le bromo-4 éthoxy-2 phénol, le bromo-2 éthoxy-4 phénol, le bromo-6 éthoxy-2 phénol, le bromo-4 diméthoxy-2,6 phénol, le bromo-4 dihydroxy-1,2 benzène, le bromo-2 dihydroxy-1,4 benzène, le bromo-3 dihydroxy-1,2 benzène, le dibromo-2,4 phénol, le tribromo-2,4,6 phénol.

5. Procédé selon l'une des revendications 1 à 4, caractérisé en ce que la phosphine utilisée répond à la formule générale (III) :

$$\underset{\displaystyle R}{\overset{|}{P}}-(Ar)_2 \qquad (III)$$

dans laquelle :
- les symboles Ar identiques ou différents représentent :
  * un radical phényle
  * un radical phényle substitué par un ou plusieurs radicaux ou atomes tels que alkyle ayant 1 à 4 atomes de carbone ; alcoxy ayant 1 à 4 atomes de carbone ; trifluorométhyle ; diméthylamino ; diéthylamino ; chlore ou fluor ;
  * un radical naphtyle
  * un radical naphtyle substitué par un ou plusieurs radicaux ou atomes tels que alkyle ayant 1 à 4 atomes de carbone ; alcoxy ayant 1 à 4 atomes de carbone ; trifluorométhyle ; diméthylamino ; diéthylamino ; chlore ou fluor ;
- R représente :
  * un radical alkyle ayant 1 à 12 atomes de carbone
  * les radicaux représentés par Ar.

6. Procédé selon l'une des revendications 1 à 5, caractérisé en ce que la phosphine utilisée répond à la formule générale (III) :

$$\underset{\displaystyle R}{\overset{|}{P}}-(Ar)_2 \qquad (III)$$

dans laquelle :
- les symboles Ar identiques ou différents représentent
  * un radical phényle
  * un radical phényle portant 1 ou 2 substituants choisis parmi les radicaux méthoxy, éthoxy, méthyle, éthyle, trifluorométhyle, diméthylamino et les atomes de chlore ou de fluor
  * un radical naphtyle
- R représente un radical alkyle ayant 1 à 4 atomes de carbone ou l'un des radicaux représentés par Ar.

7. Procédé selon l'une des revendications 5 ou 6, caractérisé en ce que les substituants que peuvent comporter les phosphines de formule (III) sur les radicaux Ar, sont situés en position para et/ou méta par rapport au carbone du cycle lié au phosphore.

8. Procédé selon l'une des revendications 1 à 7, caractérisé en ce que le phosphine de formule (III) est choisie parmi :
   - la triphényl-phosphine,
   - la tris(paraméthoxyphényl)phosphine,
   - la tris(paraméthylphényl)phosphine,
   - la tris(métaméthylphényl)phosphine,
   - la diphényl-méthyl-phosphine,
   - la diphényl-éthyl-phosphine,
   - la trinaphtyl-phosphine.

9. Procédé selon l'une des revendications 1 à 8, caractérisé en ce que le catalyseur utilisé est un métal noble finement divisé, du groupe VIII de la classification périodique des éléments tel que le palladium, le rhodium et l'iridium, ou leurs sels d'acides minéraux ou organiques.

10. Procédé selon l'une des revendications 1 à 9, caractérisé en ce que le catalyseur utilisé est choisi parmi les dérivés du palladium tels que les carboxylates, notamment les acétates, propionates, butyrates ou benzoates de palladium (II), et le chlorure palladeux.

11. Procédé selon l'une des revendications 1 à 10, caractérisé en ce que la quantité de catalyseur exprimée en moles d'atomes de métal ou en moles de dérivé métallique par mole d'halogènophénol de formule (II) est comprise entre $10^{-5}$ et $10^{-1}$ mole/mole et de préférence entre $10^{-4}$ et $10^{-2}$ mole/mole.

12. Procédé selon l'une des revendications 1 à 11, caractérisé en ce que la quantité de phosphine libre et/ou sous forme de complexe avec le catalyseur, est telle que le rapport molaire phosphine/métal noble du catalyseur soit compris entre 2 et 10.000, et de préférence compris entre 4 et 1.000.

13. Procédé selon l'une des revendications 1 à 12, caractérisé en ce que l'amine tertiaire utilisée est une amine de formule générale (IV)

$$N - (R_1)_3 \qquad (IV)$$

   dans laquelle :
   - les radicaux $R_1$, identiques ou différents, représentent des restes hydrocarbonés ayant de 1 à 20 atomes de carbone, tels que des radicaux alkyle, cycloalkyle, aryle ou hétérocyclique ;
   - deux radicaux $R_1$ forment ensemble et avec l'atome d'azote un hétérocyclique ayant 4 à 6 atomes.

14. Procédé selon la revendication 13, caractérisé en ce que l'amine tertiaire utilisée est une amine de formule générale (IV) dans laquelle
   - les symboles $R_1$ représentent un radical alkyle ayant 1 à 10 atomes de carbone et de préférence 1 à 4 atomes de carbone ou un radical cyclopentyle ou cyclohexyle ou un radical pyridinyle ;
   - deux radicaux $R_1$ forment ensemble et avec l'atome d'azote un cycle pipéridine ou pyrrolidine.

15. Procédé selon l'une des revendications 1 à 12, caractérisé en ce que l'amine tertiaire utilisée est une amine hétérocyclique.

16. Procédé selon la revendication 15, caractérisé en ce que l'amine hétérocyclique est la pyridine, la bétapicoline, l'alpha-picoline, la gamma-picoline, la diméthyl-2,6 pyridine, la diméthyl-3,4 pyridine, la diméthyl-2,4 pyridine, la quinoléine, l'isoquinoléine, la phtalazine, la naphtyridine-1,8, la quinoxaline, la quinazoline, la cinnoline, la ptéridine.

17. Procédé selon l'une des revendication 1 à 16, caractérisé en ce que la quantité d'amine tertiaire est suffisante pour neutraliser l'hydracide libéré par la réaction et en ce que de préférence la concentration en amine tertiaire dans le milieu est au moins égale à 2 moles par litre pendant la durée de la réaction.

18. Procédé selon l'une des revendications 1 à 17, caractérisé en ce que la pression utilisée est comprise entre 0,1 à 30 MPa (1 à 300 bar) et de préférence entre 1 à 15 MPa (10 à 150 bar).

19. Procédé selon l'une des revendications 1 à 18, caractérisé en ce qu'il est conduit dans un solvant inerte

dans les conditions de la réaction d'hydrocarbonylation choisi parmi des hydrocarbures aliphatiques ou cycloaliphatiques saturés tels que l'hexane ou le cyclohexane, ou aromatiques tels que le benzène, le toluène, les xylènes ; des esters tels que le benzoate de méthyle, le téréphtalate de méthyle, l'adipate de méthyle, le phtalate de dibutyle ; des esters ou éthers de polyols tels que le diacetate de tétraéthylène glycol ; des éthers cycliques tels que le tétrahydrofuranne ou le dioxanne.

**20.** Procédé selon l'une des revendications 1 à 19, caractérisé en ce que la concentration de l'halogénophénol de formule (II) mis en oeuvre, exprimée en poids d'halogénophénol par volume de solvant, est comprise entre 5 % et 50 % et de préférence entre 10 % et 40 %.

**21.** Procédé selon l'une des revendications 1 à 20, caractérisé en ce qu'en fin d'essai le mélange réactionnel est traité par une solution aqueuse d'hydroxyde de métal alcalin.

## Patentansprüche

**1.** Verfahren zur Herstellung eines Hydroxybenzaldehyds der allgemeinen Formel (I):

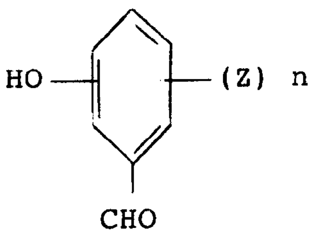

(I)

in der
- n 0, 1 oder 2 ist
- Z eine elektronenabgebende oder elektronenanziehende Gruppe ist

durch Reaktion eines Halogenphenols der allgemeinen Formel (II)

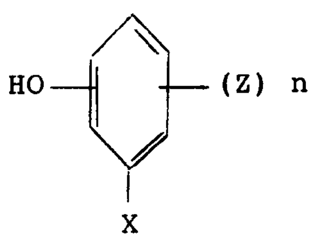

(II)

in der
- X ein Brom- oder Iodatom ist ;
- Z die obige Bedeutung hat;
- n 0, 1 oder 2 ist;

mit einem Gemisch von Kohlenmonoxid und Wasserstoff in Gegenwart eines Katalysators auf Basis eines Edelmetalls, eines tertiären Amins, eines Triarylphosphins oder eines Diarylalkylphosphins, dadurch gekennzeichnet, daß das pKa der konjugierten Säure des tertiären Amins einerseits kleiner oder gleich dem pKa des Halogenphenols der Formel (II) und andererseits größer als der des Phosphins ist.

**2.** Verfahren nach Anspruch 1, dadurch gekennzeichnet, daß man ein Halogenphenol der Formel (II) einsetzt, in dem:
- n 0, 1 oder 2 ist;
- das Symbol Z einen Hydroxylrest, ein Bromatom, ein Iodatom, einen Alkylrest, einen Alkoxyrest, einen mit einem oder mehreren Chlor- oder Fluoratomen substituierten Alkyl- oder Alkoxyrest, einen Cycloalkylrest, einen Phenylrest, einen Cycloalkoxyrest, einen Phenoxyrest, einen Alkoxycarbonylrest, einen Cycloalkoxycarbonylrest, einen Phenoxycarbonylrest, einen Alkylcarbonyloxyrest, einen Cycloalkylcarbonyloxyrest, einen Phenylcarbonyloxyrest, wobei einer der vorgenannten Reste durch ein oder mehrere Fluor- und/oder Chloratome oder Nitrilgruppen substituiert sein kann;
- das Symbol X ein Bromatom oder ein Iodatom bedeutet.

3. Verfahren nach einem der Ansprüche 1 oder 2, dadurch gekennzeichnet, daß man ein Halogenphenol der Formel (II) einsetzt, in dem
   - n 0, 1 oder 2 ist;
   - X ein Bromatom bedeutet;
   - Z die folgende Bedeutung hat:
     . einen Hydroxylrest;
     . ein Bromatom;
     . einen geradkettigen oder verzweigten Alkylrest mit 1 bis 20 Kohlenstoffatomen oder einen von einem oder mehreren Fluor- und/oder Chloratomen substituierten Alkylrest; vorzugsweise eisen niederen Alkylrest, d.h. mit 1 bis 4 Kohlenstoffatomen oder einen niederen Alkylrest, der durch 1 bis 3 Fluor- und/oder Chloratomen substituiert ist;
     . einen geradkettigen oder verzweigten Alkoxyrest mit 1 bis 20 Kohlenstoffatomen oder einen durch ein oder mehrere Fluor- und/oder Chloratome substituierten Alkoxyrest; vorzugsweise einen niederen Alkoxyrest (mit 1 bis 4 Kohlenstoffatomen) oder einen durch 1 bis 3 Fluor- und/oder Chloratomen substituierten Alkoxyrest;
     . einen Cyclopentyl-, Cyclohexyl- oder Cyclooctylrest;
     . einen Phenylrest oder einen durch 1 bis 3 Alkyl- oder niedere Alkoxyreste substituierten Phenylrest;
     . einen Alkoxycarbonylrest mit 2 bis 11 Kohlenstoffatomen, vorzugsweise 2 bis 5 Kohlenstoffatomen;
     . einen Alkoxycarbonylalkylrest, dessen Alkoxycarbonylrest der obigen Definition entspricht und dessen Alkylteil 1 bis 4 Kohlenstoffatome enthält;
     . einen Cyclopentyloxycarbonyl- oder Cyclohexyloxycarbonylrest;
     . einen Phenoxycarbonyl- oder Methylphenoxycarbonylrest;
     . einen Alkylcarbonyloxyrest mit 2 bis 11, vorzugsweise 2 bis 5 Kohlenstoffatomen;
     . einen Cyclopentanoyloxy- oder Cyclohexanoyloxyrest;
     . einen Benzoyloxy-, Methylbenzyloxy- oder Dimethylbenzoyloxyrest.

4. Verfahren nach einem der Ansprüche 1 bis 3, dadurch gekennzeichnet, daß das Halogenphenol der Formel (II) 4-Bromphenol, 2-Bromphenol, 4-Brom-2-methoxyphenol, 2-Brom-4-methoxyphenol, 6-Brom-2-methoxyphenol, 4-Brom-2-ethoxyphenol, 2-Brom-4-ethoxyphenol, 6-Brom-2-ethoxyphenol, 4-Brom-2,6-dimethoxyphenol, 4-Brom-1,2-dihydroxybenzol, 2-Brom-1,4-dihydroxybenzol, 3-Brom-1,2-dihydroxybenzol, 2,4-Dibromphenol oder 2,4,6-Tribromphenol ist.

5. Verfahren nach einem der Ansprüche 1 bis 4, dadurch gekennzeichnet, daß das verwendete Phosphin der allgemeinen Formel (III) entspricht:

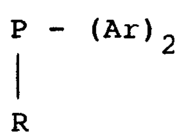

(III)

in der
   - die gleichen oder verschiedenen Symbole Ar bedeuten:
     * einen Phenylrest
     * einen durch einen oder mehrere Reste oder Atome wie Alkyl mit 1 bis 4 Kohlenstoffatomen, Alkoxy mit 1 bis 4 Kohlenstoffatomen, Trifluormethyl, Dimethylamino, Diethylamino, Chlor oder Fluor substituierten Phenylrest;
     * einen Naphtylrest
     * einen durch einen oder mehrere Reste oder Atome wie Alkyl mit 1 bis 4 Kohlenstoffatomen, Alkoxy mit 1 bis 4 Kohlenstoffatomen, Trifluormethyl, Dimethylamino, Diethylamino, Chlor oder Fluor substituierten Naphtylrest;
   - R
     * einen Alkylrest mit 1 bis 12 Kohlenstoffatomen

* die gleichen Reste wie Ar bedeutet.

6. Verfahren nach einem der Ansprüche 1 bis 5, dadurch gekennzeichnet, daß das verwendete Phosphin der allgemeinen Formel (III) entspricht :

$$\begin{array}{l} P - (Ar)_2 \\ | \\ R \end{array} \qquad (III)$$

in der
- die gleichen oder verschiedenen Symbole Ar
  * einen Phenylrest
  * einen 1 oder 2 Substituenten, ausgewählt aus den Methoxy-, Ethoxy-, Methyl-, Ethyl-, Trifluormethyl-, Dimethylaminoresten und Chlor- oder Fluoratomen,tragenden Phenylrest
  * einen Naphtylrest bedeuten
- R einen Alkylrest mit 1 bis 4 Kohlenstoffatomen oder einen der durch Ar dargestellten Reste bedeutet.

7. Verfahren nach einem der Ansprüche 5 oder 6, dadurch gekennzeichnet, daß die Substituenten, die die Phosphine der Formel (III) an den Ar-Resten tragen können, in Para- und/oder Metastellung zum Kohlenstoff des an Phosphor gebundenen Rings stehen.

8. Verfahren nach einem der Ansprüche 1 bis 7, dadurch gekennzeichnet, daß das Phosphin der Formel (III) ausgewählt ist unter:
- Triphenylphosphin,
- Tris(paramethoxyphenyl)phosphin,
- Tris(paramethylphenyl)phosphin,
- Tris(metamethylphenyl)phosphin,
- Diphenylmethylphosphin,
- Diphenylethylphosphin,
- Trinaphtylphosphin.

9. Verfahren nach einem der Ansprüche 1 bis 8, dadurch gekennzeichnet, daß der verwendete Katalysator ein fein verteiltes Edelmetall der Gruppe VIII des periodischen Systems, wie Palladium, Rhodium und Iridium, oder deren Salze von anorganischen oder organischen Säuren ist.

10. Verfahren nach einem der Ansprüche 1 bis 9, dadurch gekennzeichnet, daß der verwendete Katalysator unter den Palladiumderivaten wie den Carboxylaten, insbesondere den Acetaten, Propionaten, Butyraten oder Benzoaten des Palladiums (II), oder Palladium(II)chlorid ausgewählt ist.

11. Verfahren nach einem der Ansprüche 1 bis 10, dadurch gekennzeichnet, daß die Menge des Katalysators, ausgedrückt in Grammatomen des Metalls oder in Molen der Metallverbindung, zwischen $10^{-5}$ und $10^{-1}$, vorzugsweise zwischen $10^{-4}$ und $10^{-2}$ Mol/Mol des Halogenphenols der Formel (II) beträgt.

12. Verfahren nach einem der Ansprüche 1 bis 11, dadurch gekennzeichnet, daß die Menge des freien und-/oder in Form eines Komplexes mit dem Katalysator vorliegenden Phosphins derart ist, daß das Molverhältnis von Phosphin/Edelmetall des Katalysators zwischen 2 und 10.000, vorzugsweise zwischen 4 und 1.000 liegt.

13. Verfahren nach einem der Ansprüche 1 bis 12, dadurch gekennzeichnet, daß das verwendete tertiäre Amin ein Amin der allgemeinen Formel (IV)

$$N - (R_1)_3 \quad (IV)$$

ist, in der
- die gleichen oder verschiedenen Reste $R_1$ Kohlenwasserstoffreste mit 1 bis 20 Kohlenstoffatomen, wie Alkyl- Cycloalkyl-, Aryl- oder heterocyclische Reste bedeuten;
- zwei Reste $R_1$, zusammen mit dem Stickstoffatom einen Heterocyclus mit 4 bis 6 Atomen bilden.

14. Verfahren nach einem Anspruch 13, dadurch gekennzeichnet, daß das verwendete tertiäre Amin ein Amin

der allgemeinen Formel (IV) ist, in der

- die Symbole $R_1$ einen Alkylrest mit 1 bis 10 Kohlenstoffatomen, vorzugsweise 1 bis 4 Kohlenstoffatomen oder einen Cyclopentyl- oder Cyclohexyl- oder einen Pyridinylrest bedeuten;
- zwei Reste $R_1$, zusammen mit dem Stickstoffatom einen Piperidin- oder Pyrrolidinring bilden.

**15.** Verfahren nach einem der Ansprüche 1 bis 12, dadurch gekennzeichnet, daß das verwendete tertiäre Amin ein heterocyclisches Amins ist.

**16.** Verfahren nach Anspruch 15, dadurch gekennzeichnet, daß das heterocyclische Amin Pyridin, Beta-picolin, Alpha-picolin, Gamma-picolin, 2,6-Dimethylpyridin, 3,4-Dimethylpyridin, 2,4-Dimethylpyridin, Chinolin, Isochinolin, Phtalazin, 1,8-Naphtyridin, Chinoxalin, Chinazolin, Cinnolin oder Pteridin ist.

**17.** Verfahren nach einem der Ansprüche 1 bis 16, dadurch gekennzeichnet, daß die Menge des tertiären Amins ausreicht, um die durch die Reaktion freigesetzte Säure zu neutralisieren und daß die Konzentration des tertiären Amins in dem Milieu während der Dauer der Reaktion vorzugsweise wenigstens 2 Mol pro Liter beträgt.

**18.** Verfahren nach einem der Ansprüche 1 bis 17, dadurch gekennzeichnet, daß der verwendete Druck 0,1 bis 30 MPa (1 bis 300 bar) und vorzugsweise zwischen 1 und 15 MPa (10 bis 150 bar) beträgt.

**19.** Verfahren nach einem der Ansprüche 1 bis 18, dadurch gekennzeichnet, daß es in einem inerten Lösungsmittel unter den Bedingungen der Hydrocarbonylierungsreaktion durchgeführt wird, das ausgewählt ist unter gesättigten aliphatischen oder cycloaliphatischen Kohlenwasserstoffen wie Hexan oder Cyclohexan, oder aromatischen Kohlenwasserstoffen wie Benzol, Toluol oder Xylolen, Estern wie Methylbenzoat, Methylterephthalat, Methyladipat, Dibutylphthalat, Estern oder Ethern von Polyolen wie Tetraethylenglykoldiacetat, cyclischen Ethern wie Tetrahydrofuran oder Dioxan.

**20.** Verfahren nach einem der Ansprüche 1 bis 19, dadurch gekennzeichnet, daß die Konzentration des eingesetzten Halogenphenols der Formel (II), ausgedrückt als Gewicht des Halogenphenols pro Volumen des Lösungsmittels 5 bis 50 % und vorzugsweise 10 bis 40 % beträgt.

**21.** Verfahren nach einem der Ansprüche 1 bis 20, dadurch gekennzeichnet, daß das Reaktionsgemisch am Ende des Versuches mit der wäßrigen Lösung eines Alkalihydroxids behandelt wird.

## Claims

**1.** Process for the preparation of a hydroxybenzaldehyde of general formula (I):

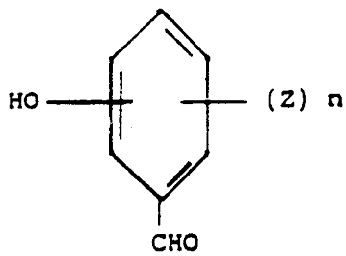

(I)

wherein

- n represents 0 or 1 or 2
- Z represents an electron-donor group or an electron-attractor group

by reaction of a halophenol of general formula (II)

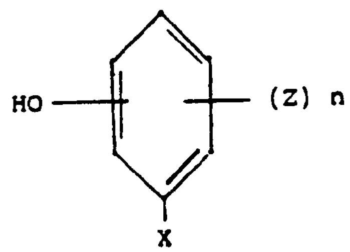

(II)

wherein

- X represents a bromine atom or an iodine atom;
- Z has the meanings given above;
- n represents 0 or 1 or 2;

with a mixture of carbon monoxide and hydrogen, in the presence of a noble metal-based catalyst, a tertiary amine, a triarylphosphine or a diarylalkylphosphine, characterised in that the tertiary amine is such that the pKa of its conjugate acid is, on the one hand lower than or equal to the pKa of the halophenol of formula (II) and, on the other hand, higher than that of the phosphine.

**2.** Process according to claim 1, characterised in that a halophenol of formula (II) is used, wherein:
- n represents 0 or 1 or 2;
- the symbol Z represents a hydroxyl radical, a bromine atom, an iodine atom, an alkyl radical, an alkoxy radical, an alkyl or alkoxy radical carrying one or more substituent chlorine or fluorine atoms, a cycloalkyl radical, a phenyl radical, a cycloalkoxy radical, a phenoxy radical, an alkoxycarbonyl radical, a cycloalkoxycarbonyl radical, a phenoxycarbonyl radical, an alkylcarbonyloxy radical, a cycloalkylcarbonyloxy radical, a phenylcarbonyloxy radical, one of the above radicals carrying one or more substituent fluorine and/or chlorine atoms or nitrile groups;
- the symbol X represents a bromine atom or an iodine atom.

**3.** Process according to one of claims 1 or 2, characterised in that a halophenol of formula (II) is used, wherein:
- n represents 0 or 1 or 2;
- X represents a bromine atom;
- Z represents:
  . a hydroxyl radical
  . a bromine atom
  . a linear or branched alkyl radical of 1 to 20 carbon atoms or an alkyl radical carrying one or more substituent fluorine and/or chlorine atoms; preferably a lower alkyl radical, i.e. one having 1 to 4 carbon atoms, or a lower alkyl radical carrying 1 to 3 substituent fluorine and/or chlorine atoms;
  . a linear or branched alkoxy radical having 1 to 20 carbon atoms or an alkoxy radical carrying one or more substituent fluorine and/or chlorine atoms; preferably a lower alkoxy radical (having 1 to 4 carbon atoms) or a lower alkoxy radical carrying 1 to 3 substituent fluorine and/or chlorine atoms;
  . a cyclopentyl, cyclohexyl or cyclooctyl radical;
  . a phenyl radical or a phenyl radical carrying 1 to 3 substituent lower alkyl or alkoxy radicals;
  . an alkoxycarbonyl radical having 2 to 11 carbon atoms and preferably 2 to 5 carbon atoms;
  . an alkoxycarbonylalkyl radical whose alkoxycarbonyl portion is as previously defined and whose alkyl portion contains 1 to 4 carbon atoms;
  . a cyclopentyloxycarbonyl or cyclohexyloxycarbonyl radical;
  . a phenoxycarbonyl or methylphenoxycarbonyl radical;
  . an alkylcarbonyloxy radical having 2 to 11 carbon atoms and preferably 2 to 5 carbon atoms;
  . a cyclopentanoyloxy or cyclohexanoyloxy radical;
  . a benzoyloxy, methylbenzoyloxy or dimethylbenzoyloxy radical.

**4.** Process according to one of claims 1 to 3, characterised in that the halophenol of formula (II) is 4-bromophenol, 2-bromophenol, 4-bromo-2-methoxyphenol, 2-bromo-4-methoxyphenol, 6-bromo-2-methoxyphenol, 4-bromo-2-ethoxyphenol,2-bromo-4-ethoxyphenol,6-bromo-2-ethoxyphenol, 4-bromo-2,6-dimethoxyphenol, 4-bromo-1,2-dihydroxybenzene, 2-bromo-1,4-dihydroxybenzene, 3-bromo-1,2-dihydroxybenzene, 2,4-dibromophenol, 2,4,6-tribromophenol.

5. Process according to one of claims 1 to 4, characterised in that the phosphine used corresponds to the general formula (III)

$$\underset{\substack{|\\ R}}{P} - (Ar)_2 \qquad (III)$$

wherein:
- the symbols Ar which may be the same or different, represent:
  * a phenyl radical
  * a phenyl radical carrying one or more substituent radicals or atoms such as alkyl having 1 to 4 carbon atoms; alkoxy having 1 to 4 carbon atoms; trifluoromethyl; dimethylamino; diethylamino; chlorine or fluorine;
  * a naphthyl radical
  * a naphthyl radical carrying one or more substituent radicals or atoms such as alkyl having 1 to 4 carbon atoms; alkoxy having 1 to 4 carbon atoms; trifluoromethyl; dimethylamino; diethylamino; chlorine or fluorine;
- R represents:
  * an alkyl radical having 1 to 12 carbon atoms
  * the radicals represented by Ar.

6. Process according to one of claims 1 to 5, characterised in that the phosphine used corresponds to the general formula (III)

$$\underset{\substack{|\\ R}}{P} - (Ar)_2 \qquad (III)$$

wherein:
- the symbols Ar which may be the same or different, represent:
  * a phenyl radical
  * a phenyl radical carrying 1 or 2 substituents selected from methoxy, ethoxy, methyl, ethyl, trifluoromethyl, dimethylamino radicals and chlorine or fluorine atoms.
  * a naphthyl radical
- R represents an alkyl radical having 1 to 4 carbon atoms or one of the radicals represented by Ar.

7. Process according to one of claims 5 or 6, characterised in that the substituents which may be carried by the Ar radicals in the phosphines of formula (III) are located in the para and/or meta positions with respect to the ring carbon linked to the phosphorus atom.

8. Process according to one of claims 1 to 7, characterised in that the phosphine of formula (III) is selected from:
- triphenylphosphine,
- tris(paramethoxyphenyl)phosphine,
- tris(paramethylphenyl)phosphine,
- tris(metamethylphenyl)phosphine,
- diphenylmethylphosphine,
- diphenylethylphosphine,
- trinaphthylphosphine.

9. Process according to one of claims 1 to 8, characterised in that the catalyst used is a finely divided noble metal, of group VIII of the Periodic Table of elements such as palladium, rhodium and iridium or their salts of inorganic or organic acids.

10. Process according to one of claims 1 to 9, characterised in that the catalyst used is selected from derivatives of palladium such as carboxylates, particularly palladium (II) acetates, propionates, butyrates, or benzoates and palladous chloride.

11. Process according to one of claims 1 to 10, characterised in that the quantity of catalyst expressed as

moles of metal atoms or as moles of metal derivative per mole of halophenol of formula (II) is between $10^{-5}$ and $10^{-1}$ mole/mole, and preferably between $10^{-4}$ and $10^{-2}$ mole/mole.

**12.** Process according to one of claims 1 to 11, characterised in that the quantity of phosphine, free and/or forming a complex with the catalyst, is such that the molar ratio of phosphine to noble metal in the catalyst is between 2 and 10,000, and preferably between 4 and 1,000.

**13.** Process according to one of claims 1 to 12, characterised in that the tertiary amine used is an amine of general formula (IV)

$$N - (R_1)_3 \quad (IV)$$

wherein:
- the radicals $R_1$ are the same or different and represent hydrocarbon residues having 1 to 20 carbon atoms, such as alkyl, cycloalkyl, aryl or heterocyclic radicals;
- two $R_1$ radicals and the nitrogen atom together form a heterocyclic ring having 4 to 6 atoms.

**14.** Process according to claim 13, characterised in that the tertiary amine used is an amine of general formula (IV) wherein:
- the symbols $R_1$ represent an alkyl radical having 1 to 10 carbon atoms and preferably 1 to 4 carbon atoms or a cyclopentyl or cyclohexyl radical or a pyridinyl radical;
- two $R_1$ radicals and the nitrogen atom together form a piperidine or pyrrolidine ring.

**15.** Process according to one of claims 1 to 12, characterised in that the tertiary amine used is a heterocyclic amine.

**16.** Process according to claim 15, characterised in that the heterocyclic amine is pyridine, beta-picoline, alpha-picoline, gamma-picoline, 2,6-dimethylpyridine, 3,4-dimethylpyridine, 2,4-dimethylpyridine, quinoline, isoquinoline, phthalazine, 1,8-naphthyridine, quinoxaline, quinazoline, cinnoline, pteridine.

**17.** Process according to one of claims 1 to 16, characterised in that the quantity of tertiary amine is enough to neutralise the hydracid released in the reaction and in that the concentration of tertiary amine in the medium is preferably equal to at least 2 moles per litre throughout the reaction.

**18.** Process according to one of claims 1 to 17, characterised in that the pressure used is between 0.1 and 30 Mpa (1 to 300 bar) and preferably 1 to 15 Mpa (10 to 150 bar).

**19.** Process according to one of claims 1 to 18, characterised in that it is carried out in a solvent that is inert under the conditions of the hydrocarbonylation reaction, selected from saturated aliphatic or cycloaliphatic hydrocarbons such as hexane or cyclohexane, or aromatic hydrocarbons such as benzene, toluene, the xylenes; esters such as methyl benzoate, methyl terephthalate, methyl adipate, dibutyl phthalate; polyol esters or ethers such as tetraethylene glycol diacetate; cyclic ethers such as tetrahydrofuran or dioxane.

**20.** Process according to one of claims 1 to 19, characterised in that the halophenol of formula (II) used, expressed as weight of halophenol per volume of solvent, is between 5 % and 50 % and preferably between 10 % and 40 %.

**21.** Process according to one of claims 1 to 20, characterised in that at the end of the experiment, the reaction mixture is treated with an aqueous solution of an alkali metal hydroxide.